# FASCICULE DE BREVET EUROPEEN

(11) **EP 0 755 917 B1**
(45) Date de publication et mention de la délivrance du brevet: **08.04.1998**
(21) Numéro de dépôt: 96401663.8
(22) Date de dépôt: 25.07.1996
(51) Int. Cl.: C07C 229/16, A61K 7/48, A61K 31/195

(54) **Dérivés de N,N'-di(aralkyl) N,N'-di(Carboxyalkyl) alkylène diamine, et de N-(aralkyl) N'-(carboxyalkyl) N,N'-di(carboxyalkyl) alkylène diamine et de N, N"-di(aralkyl)N,N',N"-tri(carboxyalkyl)dialkylène triamine et utilisation en pharmacie et en cosmétique**
N,N'-di(Aralkyl)N,N'-di(carboxyalkyl)alkylendiaminderivate, N-(Aralkyl)N'-(carboxyalkyl)N,N'-di(carboxyalkyl)alkylendiaminderivate und N,N"-di(Aralkyl)N,N',N"-tri(carboxyalkyl)dialkylentriaminderivate und ihre Verwendung in Pharmazie und Kosmetik
N,N'-di(aralkyl) N,N'-di(carboxyalkyl) alkylen- diamino-derivatives, N-(aralkyl) N'-(carboxyalkyl) N,N'-di(carboxyalkyl) alkylen- diamino-derivatives and N,N"-di(aralkyl)N,N',N"-tri(carboxyalkyl)dialkylen triamino derivatives and their use in pharmacy and cosmetics

(30) Priorité: 26.07.1995 FR 9509119
(43) Date de publication de la demande: 29.01.1997
(73) Titulaire: L'OREAL, 75008 Paris (FR)
(72) Inventeur: Galey, Jean-Baptiste, 93600 Aulnay-sous-Bois (FR); Genard, Sylvie, 75012 Paris (FR)
(74) Mandataire: Stalla-Bourdillon, Bernard

(56) Documents cités:
- EP-A- 0 367 223
- WO-A-94/11338
- US-A- 4 528 196
- INORG. CHIM. ACTA (ICHAA3,00201693);87; VOL.138 (3); PP.215-30, TEXAS A AND M UNIV.;DEP. CHEM.; COLLEGE STATION; 77843; TX; USA (US), XP000566129 MARTELL A E ET AL: "Development of iron chelators for Cooley's anemia"

## Description

La présente invention a pour objet de nouveaux dérivés de de N,N'-di(aralkyl) N,N'-di(carboxyalkyl) alkylène diamine, de N-(aralkyl) N'-(carboxyalkyl) N,N'-di(carboxyalkyl) alkylène diamine et de N,N''-di(aralkyl) N,N',N''-tri(carboxyalkyl) dialkylène triamine qui trouvent une utilisation dans des compositions pharmaceutiques et cosmétiques en vue de protéger l'organisme contre le stress oxydant.

Le stress oxydant est défini pour un certain nombre de situations physiopathologiques et pathologiques comme un déséquilibre de la balance antioxydant-prooxydant. Ce déséquilibre se traduit notamment par des processus oxydatifs non contrôlés au sein des tissus vivants qui mettent en jeu des radicaux libres oxygénés et conduisent notamment à la formation de dégâts oxydatifs sur les molécules et macromolécules biologiques.

Un certain nombre de situations physiopathologiques provoquent, favorisent, accompagnent ou sont la conséquence directe d'un stress oxydant. Il s'agit notamment de l'inflammation, du vieillissement, de l'exposition aux ultra-violets et aux rayonnements ionisants, de la carcinogénèse, des situations d'ischémie reperfusion, de la toxicité et/ou du mode d'action de certains médicaments.

Lors d'un stress oxydant, il a été montré que du fer était libéré de ses sites de stockage usuels comme la ferritine et pouvait alors participer à certaines réactions et notamment aux réactions de Fenton et Haber-Weiss dont résulte la formation des radicaux hydroxyles, radicaux connus pour être responsables de nombreux dommages oxydatifs.

Il a été proposé en vue de se protéger contre les radicaux hydroxyles d'utiliser des molécules telles que le D-mannitol, l'acide benzoïque, ou le DMSO, ceux-ci étant capables de piéger les radicaux hydroxyles. Toutefois les radicaux hydroxyles sont particulièrement réactifs et il convient d'utiliser des quantités relativement importantes de ces piégeurs de manière à entrer en compétition avec toutes les molécules biologiques, cibles potentielles des radicaux hydroxyles ce qui n'est pas sans présenter certains inconvénients du fait des problèmes de toxicité de ces piégeurs.

Par ailleurs, en vue de se protéger contre les radicaux hydroxyles, il a été proposé d'utiliser des chélateurs du fer, notamment la deferoxamine ou l'acide diéthylène triamine pentaacétique (DTPA), ceci en vue d'empêcher le fer de participer aux réactions de Fenton et Haber-Weiss.

Néanmoins il s'est avéré que la plupart des ces chélateurs sont relativement toxiques, ceux-ci pouvant interférer avec le métabolisme du fer et chelater le fer des sites actifs de certains enzymes ou d'hémoprotéines comme l'hémoglobine.

Dans la demande de brevet WO 94/11338, il a été proposé d'utiliser certains composés susceptibles de former des complexes avec le fer dont les constantes de stabilité sont faibles, ce qui diminue par conséquent les risques de toxicité associés à leur utilisation.

La demande de brevet WO 94/11338 décrit notamment l'utilisation de certains composés qui peuvent être représentés par la formule générale suivante : 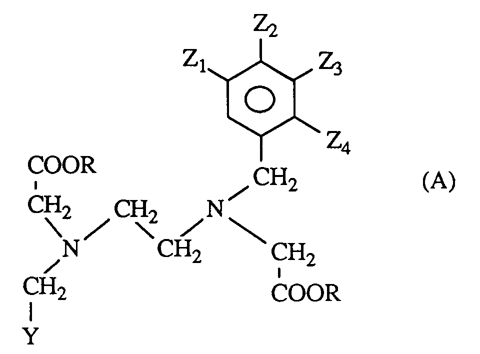 dans laquelle :
Z₁, Z₂ et Z₃, identiques ou différents, représentent un atome d'hydrogène, NO₂, COOH, CF₃, un atome d'halogène ou un groupe OR₁, SR₁ ou NR₁R₂,
R, R₁, R₂ et Z₄, identiques ou différentes, représentant un atome d'hydrogène ou un radical alkyle, linéaire ou ramifié, en C₁-C₈, et
Y représente le radical de formule 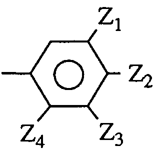 : ou le radical -COOR, et leurs sels et complexes métalliques.

Après de nouvelles recherches sur des substances agissant selon un mécanisme comparable, on a réussi la synthèse d'une nouvelle classe de composés présentant un meilleur pouvoir complexant du fer, ou une meilleure biodisponibilité, notamment une meilleure solubilité, ce qui jusqu'à présent n'avait pu être obtenu avec les complexants du fer de l'art antérieur.

On a en effet constaté de façon surprenante et inattendue qu'en augmentant dans les composés de formule (A) ci-dessus le nombre d'atomes de carbone entre les atomes d'azote et/ou en augmentant le nombre d'atomes de carbone des chaines latérales carboxylées, on obtenait des composés ayant des propriétés très nettement améliorées quant à leur biodisponibilité, celle-ci résultant essentiellement en une meilleure solubilité dans l'eau et dans les véhicules généralement utilisés dans les préparations pharmaceutiques et/ou cosmétiques.

Il s'est par ailleurs avéré que ces nouveaux composés formaient avec le fer des complexes dont les constantes d'association sont plus faibles que celles des chélateurs connus, tels que la deferoxamine. En outre, dans la mesure où, pour des raisons thermodynamiques, ils ne peuvent déplacer le fer de la transferrine, les risques toxicologiques sont diminués.

De plus le potentiel d'oxydo-réduction des complexes de fer des composés selon l'invention est tel qu'ils sont réductibles par les réducteurs physiologiques et capables sous forme réduite, de réagir avec le peroxyde d'hydrogène pour former des radicaux hydroxyles qui sont aussitôt piégés par un processus d'hydroxylation aromatique intramoléculaire de façon quasi stoechiométrique, avant qu'ils ne puissent attaquer d'autres molécules.

Après hydroxylation intramoléculaire, ces composés possèdent alors un résidu hydroxy aromatique qui peut occuper un cinquième ou un sixième site de coordination du fer. Etant donné la grande affinité des résidus phénolates pour le fer ferrique, ceci a pour conséquence d'augmenter la stabilité des complexes de plusieurs ordres de grandeur et d'empêcher la participation ultérieure du fer à la catalyse de dommages oxydatifs.

La présente invention a donc pour objet à titre de composés nouveaux, des dérivés de N,N'-di(aralkyl) N,N'-di(carboxyalkyl) alkylène diamine, de N-(aralkyl) N'-(carboxyalkyl) N,N'-di(carboxyalkyl) alkylène diamine et de N,N''-di(aralkyl) N,N',N''-tri(carboxyalkyl) dialkylène triamine pouvant être représentés par la formule générale suivante : 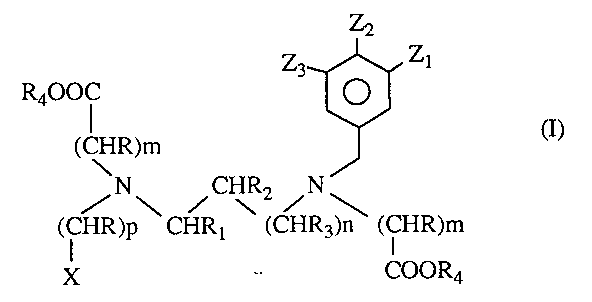 dans laquelle :
n est 0, 1 ou 2,
m et p sont 1, 2 ou 3,
R, R₁, R₂, R₃ et R₄, identiques ou différents, représentent un atome d'hydrogène ou un radical alkyle, linéaire ou ramifié, en C₁-C₄,
R₁ et R₂ ou R₂ et R₃ pris ensemble pouvant former un cycle à 5 ou 6 chaînons,
X représente le radical -COOR₄, ou le radical de formule : 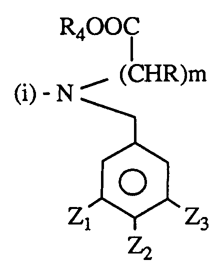 ou encore le radical de formule : 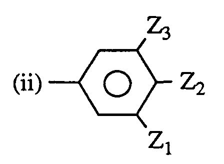 lorsque dans ce dernier cas p est égal à 1,
Z₁, Z₂ et Z₃, identiques ou différents, représentent un atome d'hydrogène, un radical alkyle, linéaire ou ramifié, en C₁-C₄, le radical -OR₅ ou -NR₅R'₅, R₅ et R'₅ représentant un atome d'hydrogène ou un radical alkyle, linéaire ou ramifié, en C₁-C₄,
   sous réserve que lorsque n=0, m=1 et X représente -COOR₄ ou le radical de formule (ii), l'un au moins des radicaux R, R₁ ou R₂ est différent d'un atome d'hydrogène,
   et leurs sels et complexes métalliques.

Par radical alkyle, linéaire ou ramifié, en C₁-C₄ on doit entendre des radicaux tels que méthyle, éthyle, isopropyle et tert-butyle.

Lorsque R₁ et R₂ ou R₂ et R₃ pris ensemble forment un cycle à 5 ou 6 chaînons, il s'agit essentiellement d'un cycle cyclopentyle ou cyclohexyle éventuellement substitué.

Selon une première forme préférée des composés selon l'invention, deux au moins des radicaux Z₁, Z₂ et Z₃ sont différents d'un atome d'hydrogène.

Selon une deuxième forme préférée des composés selon l'invention, les radicaux Z₁, Z₂ et Z₃ sont des groupes électro donneurs et de préférence représentent au moins un groupe méthoxy.

Parmi les sels des composés de formule (I) on peut notamment citer les sels d'addition d'un acide minéral tel que l'acide sulfurique, l'acide chlorhydrique, l'acide nitrique ou l'acide phosphorique ainsi que les sels d'addition d'une base minérale ou organique telle que la soude, la potasse ou la triétanolamine.

Parmi les complexes on peut citer ceux formés par addition de chlorure de zinc ou de chlorure de calcium.

A titre d'exemple on peut citer en tant que composés représentatifs des composés de formule (I) les suivants :
Acide N,N'-bis-(3,4,5 triméthoxybenzyl) 2-méthyl éthylènediamine N,N'-diacétique,
Acide N,N'-bis-(3,4,5 triméthoxybenzyl) propylène diamine N,N'-diacétique,
Acide N,N'-bis-(3,5 di-tert-butyl 4-hydroxybenzyl) 2-methyl éthylènediamine N,N'-diacétique,
Acide N,N'-bis-(3,4,5-triméthoxybenzyl) 1,2-cyclohexyldiamine N,N'-diacétique,
N,N'-bis-(3,4,5-triméthoxybenzyl) éthylènediamine N,N'-bis(2-méthyl) acétate d'éthyle,
Acide N,N'-bis-(3-hydroxy 4-méthoxybenzyl) éthylènediamine N,N'-dipropionique,
Acide N-(3,5 diméthoxybenzyl) éthylènediamine N,N',N'-tripropionique,
Acide N,N''-bis-(3,4,5-triméthoxybenzyl) diéthylène triamine N,N',N''-triacétique,
Acide N,N'-bis-(3,4,5-triméthoxybenzyl) 2-méthyl éthylènediamine N,N'-dipropionique.

La présente invention a également pour objet le procédé de préparation des composés de formule générale (I) qui peut être représenté par les schémas réactionnels I à III suivants :

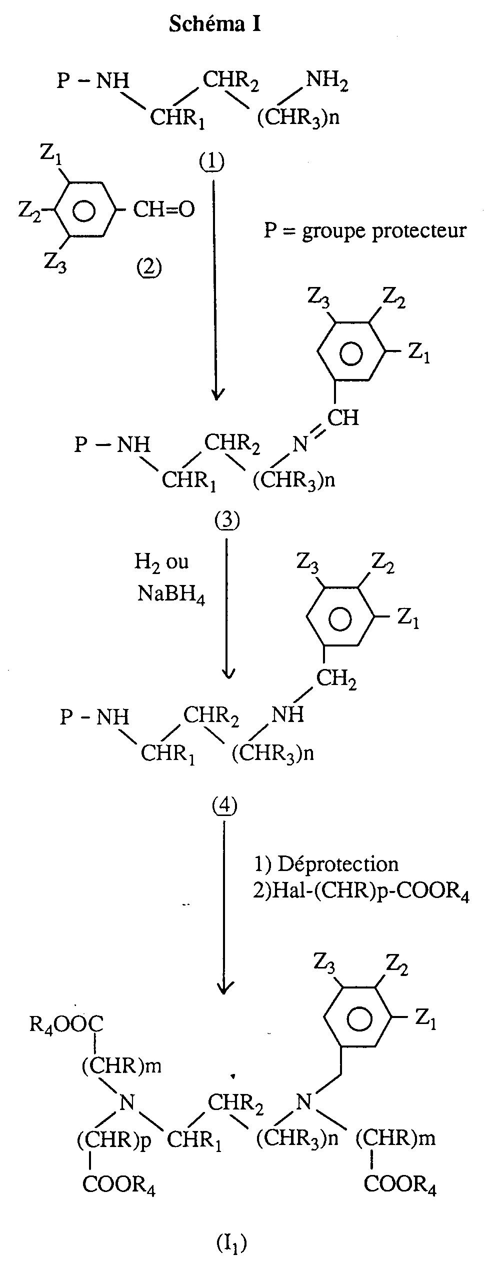

Selon le schéma I on fait réagir une alkylène diamine de formule (1), protégée par exemple par un groupe acétyle, avec 1 mole d'un aldéhyde aromatique de formule (2) en milieu solvant organique à une température inférieure au point d'ébulition du solvant. L'amino-imine (3) obtenue, isolée ou non, est ensuite réduite par exemple en présence de borohydrure de sodium ou par hydrogénation catalytique pour conduire à la diamine mono-substituée de formule (4). Cette dernière est ensuite déprotégée par HCl par exemple puis traitée en présence d'une base, à l'aide d'un halogéno acide ou ester de formule (5), (Hal étant de préférence un atome de chlore ou de brome) en une proportion de 3 moles par rapport à la diamine mono-substituée de formule (4).

Après extraction et séchage, on obtient alors le composé de formule générale (I₁) soit sous forme libre soit sous forme d'un sel d'addition d'un acide selon les conditions de traitement.

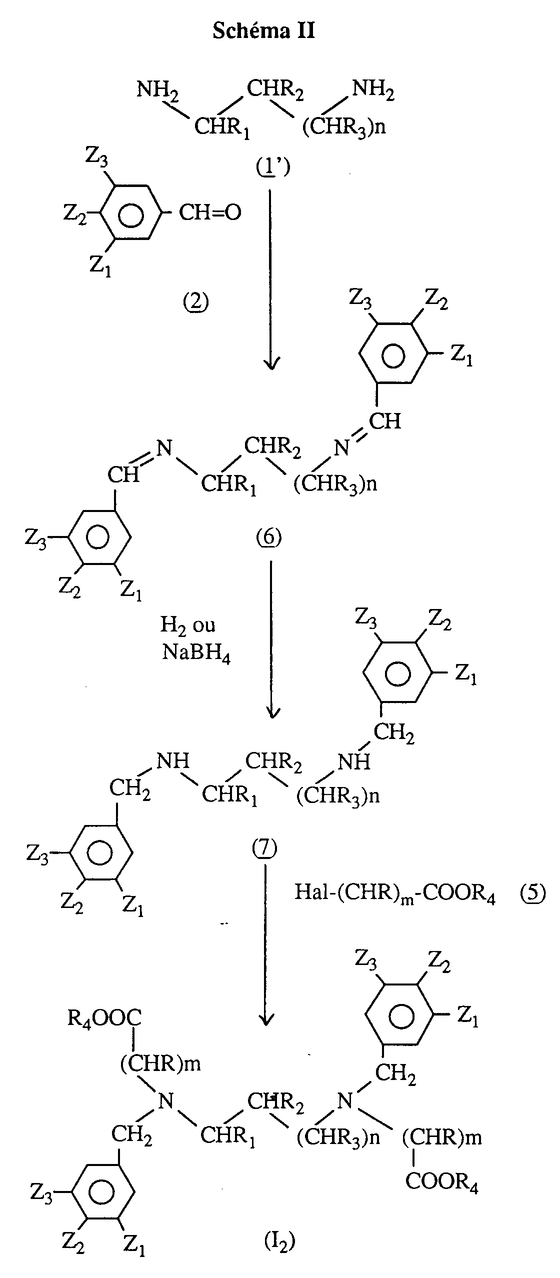

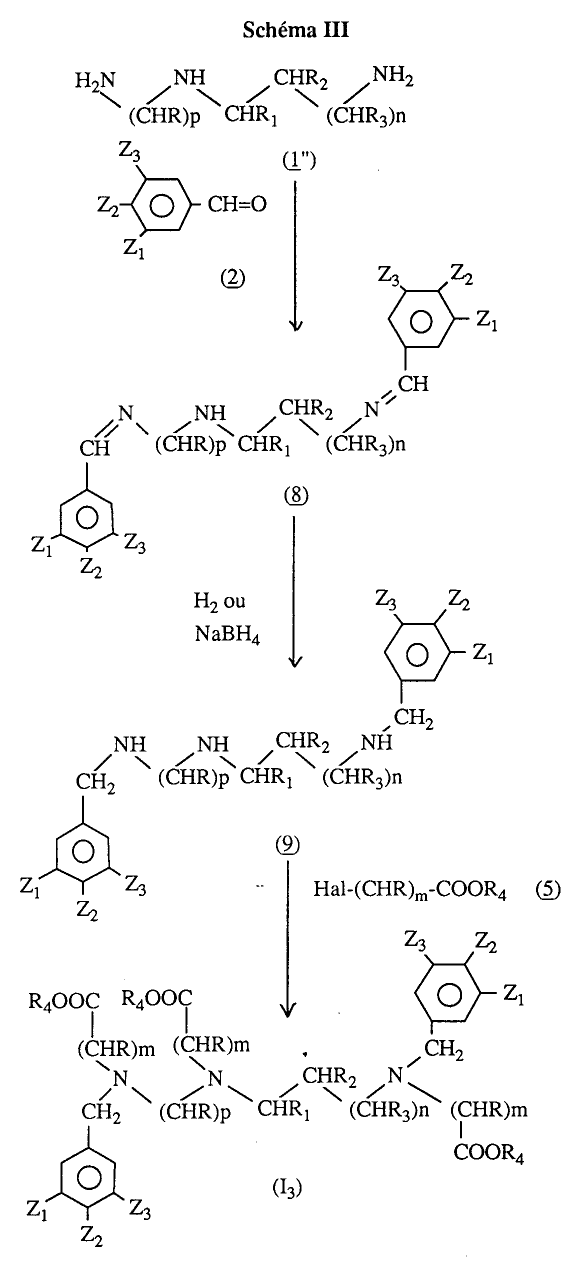

Les procédés selon les schémas II et III comprennent essentiellement les mêmes étapes que le procédé selon le schéma I sous réserve toutefois que l'on fait réagir 2 moles du composé aldéhyde aromatique pour 1 mole de l'alkylène di- ou triamine de formule (1') ou (1'') ainsi que 2 ou 3 moles de l'halogéno acide ou ester de formule (5) par rapport aux amino composés de formule (7) et (9).

La présente invention a en outre pour objet une composition cosmétique ou pharmaceutique contenant au moins un composé de formule (I) ou l'un de ses sels ou complexes métalliques dans un véhicule cosmétiquement ou pharmaceutiquement acceptable.

Dans ces compositions, le composé actif de formule (I) est généralement présent en une proportion de 0,001 à 10% en poids par rapport au poids total de la composition.

Les compositions cosmétiques peuvent se présenter sous diverses formes conventionnelles telles que sous forme d'onguent, de crème, de pommade, de gel, de spray, de lotion, d'émulsion ou de dispersion vésiculaire.

Dans les compositions selon l'invention, il a été constaté par ailleurs que le composé de formule (I) jouait un rôle important par son action antioxydante et permettait ainsi de les protéger de l'oxydation.

Lorsque le composé de formule (I) est utilisé dans le cadre d'un traitement pharmaceutique, les formes d'administration peuvent être par voie orale, topique ou parentérale, le support pharmaceutiquent acceptable étant fonction de la forme d'administration choisie. Les doses d'administration sont généralement comprises entre 1 mg et 1000 mg/kg/jour.

Les compositions pharmaceutiques selon l'invention sont tout particulièrement destinées à traiter les situations de stress oxydant liées à certains états pathologiques et notamment les maladies neurodégénératives telles que par exemple la maladie de Parkinson, les situations inflammatoires chroniques, le syndrome d'ischémie reperfusion, la toxicité de certains médicaments tels que certains xénobiotiques et les surcharges en fer.

Dans les compositions selon l'invention le composé de formule (I) peut, selon une forme de réalisation préférée, être associée à au moins une autre substance active (ou une autre substance anti-radicaux-libres). Ces substances peuvent être choisies plus particulièrement parmi :
- les antilipoperoxydants comme la vitamine E, le trolox, le BHT (butylhydroxytoluène),
- les réducteurs biologiques comme le gultathion réduit et ses dérivés, la vitamine C et ses dérivés,
- les quencheurs d'oxygène singulet comme le β-carotène,
- les systèmes capables de décomposer le peroxyde d'hydrogène et notamment des enzymes comme la catalase ou des peroxydases en présence de leurs co-substrats,
- les systèmes de protection contre l'anion superoxyde comme la superoxyde dismutase (SOD) ou des SOD-like tels que le complexe Mn-desferal ou le di-isopropyl salicylate de cuivre,
- les systèmes capables de décomposer les hydroperoxydes organiques comme la glutathion peroxydase ou des systèmes à base de sélénium.

Les composés de formule (I) et les substances actives ou substances antiradicaux libres telles que définies ci-dessus, peuvent être associés au sein de la même composition ou être appliqués séparément.

Les exemples ci-après sont donnés en vue d'illustrer le procédé de préparation des composés de formule (I) et leur utilisation dans les domaines pharmaceutique et cosmétique.

### EXEMPLES DE PREPARATION DES COMPOSES

### EXEMPLE 1 : Préparation de l'acide N,N'-bis-(3,4,5-triméthoxybenzyl) 2-méthyl éthylènediamine diacétique

1) 40 mmoles de 3,4,5 triméthoxybenzaldéhyde sont solubilisés dans 30 ml de méthanol. On additionne 20 mmoles de 1,2-diaminopropane puis le mélange est chauffé 30 minutes à 50°C. Le mélange réactionnel est concentré sous vide puis directement mis en réaction pour la deuxième étape.
2) 17 mmoles de l'imine obtenue en 1) ci-dessus sont mises en suspension dans 100 ml d'éthanol absolu. On additionne peu à peu 1 équivalent de borohydrure de sodium et on agite le mélange 1 heure à température ambiante. Après évaporation, on ajoute 20 ml d'eau au résidu et on ramène le pH à 2 par addition d'acide chlorhydrique. Le précipité est ensuite filtré, lavé à l'eau puis séché.
3) On solubilise séparément 10 mmoles de la diamine obtenue en 2) ci-dessus dans 15 ml d'eau contenant 10 mmoles d'hydroxyde de sodium et 20 mmoles d'acide bromoacétique dans 25 ml d'eau à 0°C contenant 20 mmoles de monohydrogénocarbonate de sodium.

Les deux solutions sont mélangées et chauffées à 40°C pendant 6 heures en maintenant le pH vers 12 par addition de soude à 30 %.

Le mélange est ensuite acidifié par de l'acide chlorhydrique concentré jusqu'à pH 4-5. La solution est concentrée sous vide et le précipité obtenu est filtré. On récupère l'acide N,N'-bis(3,4,5-triméthoxybenzyl) 2-méthyl éthylènediamine diacétique sous forme d'une poudre blanche de point de fusion = 144°C et de spectre RMN ¹H et d'analyse élémentaire conformes à la structure attendue.

### EXEMPLE 2 : Préparation de l'acide N,N''-bis-(4,5-triméthoxybenzyl) diéthylènetriamine N,N',N''-triacétique

1) 60 mmoles de 3,4,5 triméthoxybenzaldéhyde sont solubilisés dans 100 ml de toluène anhydre. On additionne 20 mmoles de diéthylènetriamine puis le mélange est porté à reflux dans un tricol pourvu d'un appareil de distillation de type Dean Stark jusqu'à ce que la quantité théorique d'eau ait été distillée. Le mélange réactionnel est alors évaporé à sec puis aussitôt mis en réaction pour la deuxième étape.
2) L'huile brute obtenue en 1) est solubilisée dans 100 ml d'éthanol absolu, puis l'on additionne peu à peu 4 équivalents de borohydrure de sodium et on agite le mélange 1 heure à 50°C. Après évaporation, on ajoute 20 ml d'eau au résidu et on ramène le pH à 1 par addition d'acide chlorhydrique. Le précipité est filtré, lavé puis séché. Le spectre RMN ¹H est conforme à la structure de la triamine attendue (redement sur les deux étapes 77 %).
3) 10 mmoles de la triamine obtenue en 2) sont solubilisés dans 150 ml de dichlorométhane contenant 60 mmoles de triéthylamine. 90 mmoles de bromoacétate de tertbutyle sont additionnés dans 250 ml de dichlorométhane et le mélange réactionnel est chauffé au reflux pendant 18 heures. Le mélange est ensuite refroidi, puis lavé par une solution diluée d'acide chlorhydrique puis par une solution diluée de soude. La phase organique est séchée puis concentrée sous vide. Le résidu huileux est purifié par chromatographie sur silice. On récupère une huile incolore dont le spectre RMN ¹H est conforme à la structure du triester terbutylique de l'acide N,N''-di(3,4,5-triméthoxybenzyl diéthylènetriamine N,N'N''-triacétique.
4) 1 g du triester obtenu en 3) est porté au reflux dans 100 ml d'acide chlorhydrique 1M pendant 1 heure. Le mélange réactionnel est alors évaporé à sec puis lavé à l'éthanol froid et filtré puis séché. On récupère un produit cristallisé blanc dont le spectre RMN ¹H et l'analyse élémentaire sont conformes à la structure de l'acide N,N''-di(3,4,5-triméthoxybenzyl) diéthylènetriamine N,N'N''-triacétique.

### EXEMPLE 3 : Préparation de l'acide N,N'-bis-(3,4,5-triméthoxybenzyl) 1,3-diaminopropane diacétique

Par un procédé analogue à celui décrit à l'exemple 1, on prépare l'acide N,N'-bis-(3,4,5-triméthoxybenzyl) 1,3-diaminopropane diacétique en partant du 1,3-diaminopropane. Le spectre RMN du proton, ainsi que l'analyse élémentaire sont conformes à la structure attendue.

### EXEMPLE 4 : Préparation de l'acide N,N'-bis-(3,4,5-triméthoxybenzyl) 1,2-cyclohexyldiamine N,N'diacétique

Par un procédé analogue à celui décrit à l'exemple 1, on prépare l'acide N,N'-bis-(3,4,5-triméthoxybenzyl) 1,2-cydohexyldiamine N,N- diacétique en partant de la 1,2-cyclohexyldiamine. On obtient un solide blanc de point de fusion = 150°C (dec.) dont le spectre RMN ¹H (400 MHz) et l'analyse élémentaire sont conformes à la strucutre attendue (sous forme d'un mélange contenant 1 H₂O, 0,3 HCl et 0,8 HBr).

| | C | H | N | O | Cl | Br |
|---|---|---|---|---|---|---|
| Calculé % | 52,65 | 6,60 | 4,10 | 16,38 | 1,56 | 9,36 |
| Trouvé % | 52,69 | 6,81 | 4,03 | 25,74 | 1,42 | 9,34 |

### EXEMPLE 5 : Préparation de l'acide N,N'-bis-(3,4,5-triméthoxybenzyl) 2-méthyl éthylènediamine N,N'-dipropionique

### (a) Diester éthylique de l'acide éthylènediamine N,N'-dipropionique

On additionne goutte à goutte 3,9 g de chlorure de thionyle à une suspension de 1,5 g de chlorhydate d'acide éthylènediamine N,N'-dipropionique dans 100 ml d'éthanol absolu. Le mélange est porté à 75°C pendant 24 heures. On filtre à chaud puis on refroidit les eaux mères. Le solide formé est filtré puis lavé à l'éthanol. Après recristallisation dans 15 ml d'éthanol, on obtient 1,2 g d'un solide blanc dont le spectre RMN ¹H est conforme à la structure attendue (sous forme de dichlorhydrate).

### (b) Diester éthylique de l'acide N,N'-bis-(3,4,5-triméthoxybenzyl) éthylènediamine N,N'-dipropionique

On chauffe à 80°C pendant 4 heures un mélange de 1 g de dichlorhydrate du diester éthylique de l'acide éthylènediamine N,N'-dipropionique, 0,9 ml de triéthylamine, 0,75 g de CaCO₃ et 1,6 g de chlorure de 3,4,5-triméthoxybenzyle. Après refroidissement, on évapore à sec le mélange et on le reprend dans 50 ml d'eau. La solution est acidifiée jusqu'à pH 1 avec HCl concentré puis extraite par 3 x 25 ml de dichlorométhane. La phase organique est lavée à l'eau saturée de NaCl puis séchée et évaporée à sec. L'huile obtenue est purifiée par chromatographie sur colonne de silice (éluant dichlorométhane/méthanol 98:2). On obtient 0,6 g d'une huile dont le spectre RMN ¹H (400 MHz) est en accord avec la structure du diester éthylique de l'acide N,N'-bis-(3,4,5-triméthoxybenzyl) éthylènediamine N,N'-dipropionique.

### (c) Acide N,N'-bis-(3,4,5-triméthoxybenzyl) 2-méthyl éthylènediamine N,N'-dipropionique

On solubilise 2,2 g de diester éthylique de l'acide N,N'-bis-(3,4,5-triméthoxybenzyl) éthylènediamine N,N'-dipropionique dans un mélange de 20 ml de NaOH 1N et 40 ml de méthanol. Le mélange est agité 2 heures à température ambiante.

La solution est mise à sec puis reprise par 25 ml d'eau.

Après lavage par 3 x 25 ml de dichlorométhane, la phase aqueuse est acidifiée à pH 4,5 par HCl concentré puis évaporée à sec. On additionne alors 25 ml d'éthanol et le mélange est refroidi à 0°C. Les sels sont éliminés par filtration, puis la solution est concentrée jusqu'à un volume de 5 ml. On additionne alors 0,5 ml d'HCl 0,8 N et le mélange est agité pendant 1 heure. Le solide apparu est alors filtré puis rincé à l'éther éthylique.

On obtient 200 mg d'une poudre blanche de point de fusion = 220°C (dec.) dont le spectre RMN ¹H (400 MHz) et l'analyse élémentaire sont conformes à la structure attendue (sous forme de dichlorhydrate).

| | C | H | N | O | Cl |
|---|---|---|---|---|---|
| Calculé % (avec 2 H₂O et 0,7 NaCl) | 47,06 | 6,44 | 3,92 | 26,89 | 13,43 |
| Trouvé % | 46,95 | 6,54 | 3,94 | 26,41 | 12,33 |

### TESTS DE SOLUBILITE COMPARATIVE

| Composés | Solvant |
|---|---|
| | Tampon Acétate 50 mM pH 5,8 |
| Acide N,N'-bis-(3,4,5-triméthoxybenzyl) 2-méthyl éthylènediamine N,N'-diacétique, 2 HCl | > 10 % |
| Acide N,N'-bis-(3,4,5-triméthoxybenzyl) éthylènediamine N,N'-diacétique, 2 HCl (Composé selon WO 94/11338) | < 0,5 % |

### EXEMPLES DE FORMULATION

### I - COSMETIQUE

### EXEMPLE A

On prépare une émulsion selon les techniques classiques à l'aide des constituants suivants :

| | |
|---|---|
| - Acide N,N'-bis-(3,4,5-triméthoxybenzyl) 2-méthyl éthylènediamine diacétique (composé de l'exemple 1) | 0,1 g |
| - Polyéthylèneglycol oxyéthyléné à 50 moles | 3,0 g |
| - Stéarate de mono/diglycéryle | 3,0 g |
| - Huile de vaseline | 24,0 g |
| - Alcool cétylique | 5,0 g |
| - Eau qsp | 100,0 g |

### EXEMPLE B

On prépare une émulsion selon les techniques classiques à l'aide des constituants suivants :

| | |
|---|---|
| - Acide N,N'-bis-(3,4,5-triméthoxybenzyl) 2-méthyl éthylènediamine diacétique (composé de l'exemple 1) | 0,5 g |
| - Palmitate d'octyle | 10,0 g |
| - Isostéarate de glycéryle | 4,0 g |
| - Huile de vaseline | 24,0 g |
| - Vitamine E | 1,0 g |
| - Glycérol | 3,0 g |
| - Eau qsp | 100,0 g |

### EXEMPLE C

On prépare la composition suivante selon les techniques classiques à l'aide des constituants suivants :

| | |
|---|---|
| - Acide N,N'-bis-(3,4,5-triméthoxybenzyl) 2-méthyl éthylènediamine diacétique (composé de l'exemple 1) | 0,10 g |
| - Huile de jojoba | 13,00 g |
| - Sorbate de potassium | 0,30 g |
| - Cyclopentadimethylsiloxane | 10,00 g |
| - Alcool stéarylique | 1,00 g |
| - Acide stéarique | 4,00 g |
| - Stéarate de polyéthylène glycol | 3,00 g |
| - Vitamine E | 1,00 g |
| - Glycérol | 3,00 g |
| - Conservateurs | 0,05 g |
| - Eau qsp | 100,00 g |

Les compositions des exemples A à C ci-dessus appliquées régulièrement une fois par jour, de préférence le soir, permettent de prévenir de façon particulièrement significative le vieillissement cutané. Par ailleurs, on a constaté que les compositions présentaient une excellente stabilité dans le temps, le composé actif présent permettant de les protéger des phénomènes d'oxydation.

Dans ces compositions, le composé de l'exemple 1 peut être avantageusement remplacé par la même quantité d'un des composés des exemples 2, 3, 4 et 5.

### II - PHARMACEUTIQUE

### EXEMPLE A : Voie orale

| **1) Comprimé** | |
|---|---|
| - Acide N,N'-bis-(3,4,5-triméthoxybenzyl) 2-méthyl éthylènediamine diacétique (composé de l'exemple 1) | 0,001 g |
| - Amidon | 0,114 g |
| - Phosphate bicalcique | 0,020 g |
| - Lactose | 0,060 g |
| - Stéarate de magnésium | 0,005 g |

| **2) Suspension buvable** | |
|---|---|
| - Acide N,N'-bis-(3,4,5-triméthoxybenzyl) 2-méthyl éthylènediamine diacétique (composé de l'exemple 1) | 0,001 g |
| - Glycérol | 0,500 g |
| - Sorbitol à 70 % | 0,520 g |
| - Saccharinate de sodium | 0,010 g |
| - p-hydroxybenzoate de méthyle | 0,040 g |
| - Arôme qs | |
| - Eau purifiée qsp | 5 ml |

| **3) Suspension buvable** | |
|---|---|
| - Acide N,N''-bis-(3,4,5-triméthoxybenzyl) diéthylènetriamine N,N',N''-triacétique (composé de l'exemple 2) | 0,100 g |
| - Ethanol à 90 % | 1,000 g |
| - Sorbitol à 70 % | 0,500 g |
| - Saccharinate de sodium | 0,010 g |
| - p-hydroxybenzoate de méthyle | 0,040 g |
| - Arôme qs | |
| - Eau purifiée qsp | 5 ml |

Les formulations 1) à 3) ci-dessus administrées à une dose appropriée 1 à 2 fois par jour pendant environ 3 à 5 semaines, permettent de traiter de façon efficace la plupart des maladies neurodégénératives.

Dans les formulations 1) et 2), le composé de l'exemple 1 peut être avantageusement remplacé par la même quantité d'un des composés des exemples 3, 4 et 5.

Dans la formulation 3) le composé de l'exemple 2 peut être avantageusement remplacé par la même quantité d'un des composés des exemples 1, 4 et 5.

### EXEMPLE B : Administration par injection

| **Ampoule injectable de 3 ml** | |
|---|---|
| - Acide N,N'-bis-(3,4,5-triméthoxybenzyl) 2-méthyl éthylènediamine diacétique (composé de l'exemple 1) | 0,005 g |
| - Bicarbonate de sodium | 0,001 g |
| - Eau pour préparation injectable qsp | 3 ml |

Dans cet exemple de formulation sous forme d'une ampoule injectable, le composé selon l'exemple 1 peut être avantageusement remplacé par la même quantité d'un des composés des exemples 2, 3 et 5.

## Revendications

1. Nouveaux dérivés de N,N'-di(aralkyl) N,N'-di(carboxyalkyl) alkylène diamine, de N-(aralkyl) N'-(carboxyalkyl) N,N'-di(carboxyalkyl) alkylène diamine et de N,N''-di(aralkyl) N,N',N''-tri(carboxyalkyl) dialkylène triamine caractérisés par le fait qu'ils correspondent à la formule générale suivante : 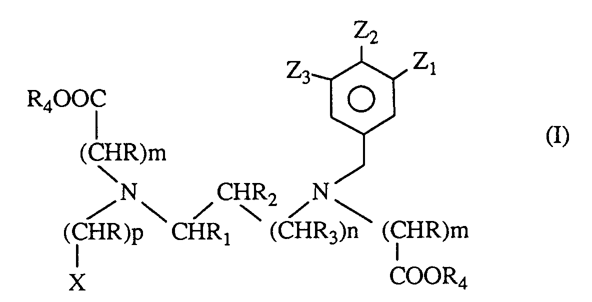 dans laquelle :
n est 0, 1 ou 2,
m et p sont 1, 2 ou 3,
R, R₁, R₂, R₃ et R₄, identiques ou différents, représentent un atome d'hydrogène ou un radical alkyle, linéaire ou ramifié, en C₁-C₄,
R₁ et R₂ ou R₂ et R₃ pris ensemble pouvant former un cycle à 5 ou 6 chaînons,
X représente le radical -COOR₄, ou le radical de formule : 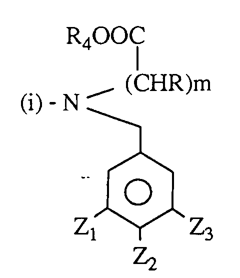 ou encore le radical de formule : 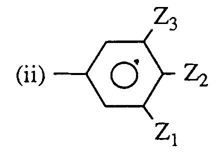 lorsque dans ce dernier cas p est égal à 1,
Z₁, Z₂ et Z₃, identiques ou différents, représentent un atome d'hydrogène, un radical alkyle, linéaire ou ramifié, en C₁-C₄, le radical -OR₅ ou -NR₅R'₅, R₅ et R'₅ représentant un atome d'hydrogène ou un radical alkyle, linéaire ou ramifié, en C₁-C₄,
sous réserve que lorsque n=0, m=1 et X est -COOR₄ ou le radical de formule (ii) l'un au moins des radicaux R, R₁ ou R₂ est différent d'un atome d'hydrogène,
et leurs sels et complexes métalliques.

2. Composés selon la revendication 1, caractérisés par le fait que le radical alkyle, linéaire ou ramifié, est choisi parmi les radicaux méthyle, éthyle, isopropyle et tert-butyle.

3. Composés selon la revendication 1 ou 2, caractérisés par le fait que deux au moins des radicaux Z₁, Z₂ et Z₃ sont différents d'un atome d'hydrogène.

4. Composés selon l'une quelconque des revendications précédentes, caractérisés par le fait que l'un au moins des radicaux Z₁, Z₂ et Z₃ représente un groupe méthoxy.

5. Composés selon l'une quelconque des revendications précédentes, caractérisés par le fait que les sels sont des sels d'addition d'un acide minéral choisi parmi l'acide sulfurique, l'acide chlorhydrique, l'acide nitrique et l'acide phosphorique.

6. Composés selon l'une quelconque des revendications 1 à 4, caractérisés par le fait que les sels sont des sels d'addition d'une base minérale ou organique choisie parmi la soude, la potasse et la triéthanolamine.

7. Composés selon l'une quelconque des revendications 1 à 4, caractérisés par le fait que les complexes sont des complexes formés par addition de chlorure de zinc ou de chlorure de calcium.

8. Composés selon l'une quelconque des revendications précédentes, caractérisés par le fait qu'ils sont choisis parmi :
Acide N,N'-bis-(3,4,5 triméthoxybenzyl) 2-méthyl éthylènediamine N,N'-diacétique,
Acide N,N'-bis-(3,4,5 triméthoxybenzyl) propylène diamine N,N'-diacétique,
Acide N,N'-bis-(3,5 di-tert-butyl 4-hydroxybenzyl) 2-methyl éthylènediamine N,N'-diacétique,
Acide N,N'-bis-(3,4,5-triméthoxybenzyl) 1,2-cydohexyldiamine N,N'-diacétique,
N,N'-bis-(3,4,5-triméthoxybenzyl) éthylènediamine N,N'-di(2-méthyl) acétate d'éthyle,
Acide N,N'-bis-(3-hydroxy 4-méthoxybenzyl) éthylènediamine N,N'-dipropionique,
Acide N-(3,5 diméthoxybenzyl) éthylènediamine N,N',N'-tripropionique,
Acide N,N''-bis-(3,4,5-triméthoxybenzyl) diéthylène triamine N,N',N''-triacétique,
Acide N,N'-bis-(3,4,5-triméthoxybenzyl) 2-méthyl éthylènediamine N,N'-dipropionique.

9. Composition pharmaceutique ou cosmétique caractérisée par le fait qu'elle contient dans un véhicule pharmaceutiquement ou cosmétiquement acceptable au moins un composé de formule (I) tel que revendiqué selon l'une quelconque des revendications 1 à 8.

10. Composition selon la revendication 9, caractérisée par le fait qu'elle contient le composé de formule (I) en une proportion de 0,001 à 10 % en poids par rapport au poids total de la composition.

11. Composition selon la revendication 9 ou 10, caractérisée par le fait qu'elle contient en outre au moins une substance active choisie parmi les antilipoperoxydants, les réducteurs biologiques, les quencheurs d'oxygène, les enzymes, une superoxyde dismutase (SOD) ou une SOD-like, la glutathion peroxydase ou des systèmes à base de sélénium.

12. Utilisation d'un composé de formule (I) selon l'une quelconque des revendications 1 à 8, pour la préparation d'une composition pharmaceutique destinée au traitement de situations de stress oxydant liées à certains états pathologiques.

13. Utilisation d'un composé de formule (I) selon l'une quelconque des revendications 1 à 8, dans et/ou pour la préparation d'une composition cosmétique ou pharmaceutique destinée à prévenir le vieillissement cutané.

14. Utilisation d'un composé de formule (I) selon l'une quelconque des revendications 1 à 8, en tant qu'agent anti-oxydant dans des compositions pharmaceutiques et cosmétiques.

## Claims

1. Novel N,N'-di(aralkyl)-N,N'-di(carboxyalkyl)alkylenediamine, N-(aralkyl)-N'-(carboxyalkyl)-N,N'-di(carboxyalkyl)alkylenediamine and N,N''-di(aralkyl)-N,N',N''-tri(carboxyalkyl)dialkylenetriamine derivatives, characterized in that they correspond to the following general formula: 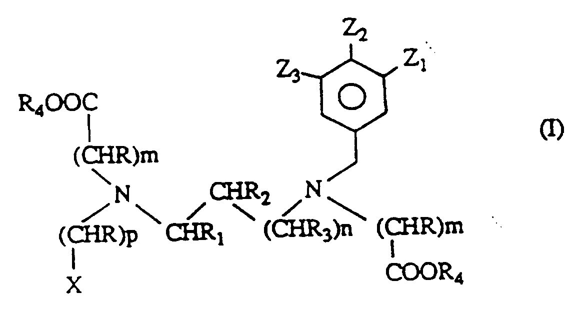 in which:
n is 0, 1 or 2,
m and p are 1, 2 or 3,
R, R₁, R₂, R₃ and R₄, which may be identical or different, represent a hydrogen atom or a linear or branched C₁-C₄ alkyl radical,
R₁ and R₂ or R₂ and R₃, taken together, being able to form a 5- or 6-membered ring,
X represents a radical -COOR₄ or a radical of formula: 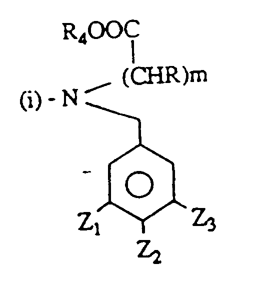 or a radical of formula: 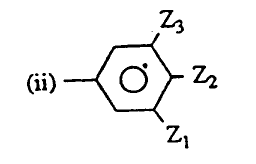 when, in the latter case, p is equal to 1,
Z₁, Z₂ and Z₃, which may be identical or different, represent a hydrogen atom, a linear or branched C₁-C₄ alkyl radical, a radical -OR₅ or -NR₅R'₅, R₅ and R'₅ representing a hydrogen atom or a linear or branched C₁-C₄ alkyl radical,
with the proviso that when n=0, m=1 and X is -COOR₄ or a radical of formula (ii), at least one of the radicals R, R₁ or R₂ is other than a hydrogen atom,
and the salts and metal complexes thereof.

2. Compounds according to Claim 1, characterized in that the linear or branched alkyl radical is chosen from the methyl, ethyl, isopropyl and tert-butyl radicals.

3. Compounds according to Claim 1 or 2, characterized in that at least two of the radicals Z₁, Z₂ and Z₃ are other than a hydrogen atom.

4. Compounds according to any one of the preceding claims, characterized in that at least one of the radicals Z₁, Z₂ and Z₃ represents a methoxy group.

5. Compounds according to any one of the preceding claims, characterized in that the salts are addition salts with an inorganic acid chosen from sulphuric acid, hydrochloric acid, nitric acid and phosphoric acid.

6. Compounds according to any one of Claims 1 to 4, characterized in that the salts are addition salts with an inorganic or organic base chosen from sodium hydroxide, potassium hydroxide and triethanolamine.

7. Compounds according to any one of Claims 1 to 4, characterized in that the complexes are complexes formed by addition of zinc chloride or calcium chloride.

8. Compounds according to any one of the preceding claims, characterized in that they are chosen from:
N,N'-bis(3,4,5-trimethoxybenzyl)-2-methylethylenediamine-N,N'-diacetic acid,
N,N'-bis(3,4,5-trimethoxybenzyl)propylenediamine-N,N'-diacetic acid,
N,N'-bis(3,5-di-tert-butyl-4-hydroxybenzyl)-2-methylethylenediamine-N,N'-diacetic acid,
N₁N'-bis(3,4,5-trimethoxybenzyl)-1,2-cyclohexyldiamine-N,N'-diacetic acid,
ethyl N,N'-bis(3,4,5-trimethoxybenzyl)ethylenediamine-N,N'-di(2-methyl) acetate,
N,N'-bis(3-hydroxy-4-methoxybenzyl)ethylenediamine-N,N'-dipropionic acid,
N-(3,5-dimethoxybenzyl)ethylenediamine-N,N',N'-tripropionic acid,
N,N''-bis(3,4,5-trimethoxybenzyl)diethylenetriamine-N,N',N''-triacetic acid,
N,N'-bis(3,4,5-trimethoxybenzyl)-2-methylethylenediamine-N,N'-dipropionic acid.

9. Pharmaceutical or cosmetic composition, characterized in that it contains, in a pharmaceutically or cosmetically acceptable vehicle, at least one compound of formula (I) as claimed according to any one of Claims 1 to 8.

10. Composition according to Claim 9, characterized in that it contains the compound of formula (I) in a proportion of from 0.001 to 10% by weight relative to the total weight of the composition.

11. Composition according to Claim 9 or 10, characterized in that it also contains at least one active substance chosen from anti-lipoperoxidizing agents, biological reducing agents, oxygen quenchers, enzymes, superoxide dismutase (SOD) or an SOD-like substance, glutathione peroxidase or selenium-based systems.

12. Use of a compound of formula (I) according to any one of Claims 1 to 8, for the preparation of a pharmaceutical composition intended for the treatment of oxidative stress situations associated with certain pathological states.

13. Use of a compound of formula (I) according to any one of Claims 1 to 8, in and/or for the preparation of a cosmetic or pharmaceutical composition intended for preventing ageing of the skin.

14. Use of a compound of formula (I) according to any one of Claims 1 to 8, as an antioxidant in pharmaceutical and cosmetic compositions.

## Patentansprüche

1. Neue Derivate des N,N'-Di-(aralkyl)-N,N'-di-(carboxyalkyl)-alkylendiamins, N-(Aralkyl)-N'-(carboxyalkyl)-N,N'-di-(carboxyalkyl)-alkylendiamins sowie des N,N''-Di-(aralkyl)-N,N'N''-tri-(carboxyalkyl)-dialkylentriamins, dadurch gekennzeichnet, daß sie der folgenden allgemeinen Formen entsprechen: 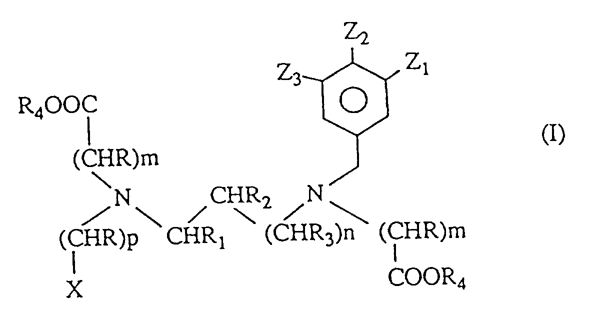 worin:
n die Zahl 0, 1 oder 2, und
m und p die Zahl 1, 2 oder 3 bedeuten,
R, R₁, R₂, R₃ und R₄, die gleich oder verschieden sein können, ein Wasserstoffatom oder einen gerad- oder verzweigtkettigen C₁-C₄-Alkylrest bedeuten, wobei
R₁ und R₂ oder R₂ und R₃ zusammengenommen einen 5- oder 6-gliedrigen Ring bedeuten können,
X den Rest -COOR₄ oder einen Rest gemäß der folgenden Formel darstellt: 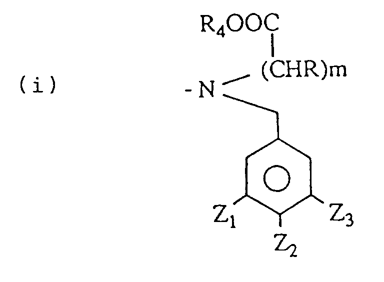 oder auch den folgenden Rest bedeutet: 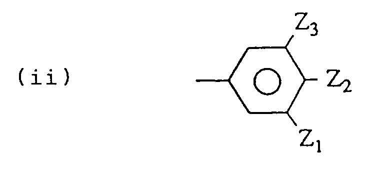 wobei in dem Falle, daß bei letztgenannter Substitution m = 1 ist,
Z₁, Z₂ und Z₃, die gleich oder verschieden sein können, ein Wasserstoffatom, einen gerad- oder verzweigtkettigen C₁-C₄-Alkylrest, den Rest -OR₅ oder -NR₅R'₅ bedeuten,
wobei R₅ und R'₅ ein Wasserstoffatom oder einen gerad- oder verzweigtkettigen C₁-C₄-Alkylrest darstellen kann, und in dem Falle, daß n = 0, m = 1 und X den Rest -COOR₄ oder den Rest gemäß der Formel (ii) bedeutet, mindestens einer der Reste R, R₁ und R₂ von einem Wasserstoffatom verschieden ist, sowie
deren Salze und Metallkomplexe.

2. Verbindungen nach Anspruch 1, dadurch gekennzeichnet, daß der gerad- oder verzweigtkettige Alkylrest unter den Gruppen Methyl, Ethyl, Isopropyl und tert-Butyl ausgewählt ist.

3. Verbindungen nach Anspruch 1 oder 2, dadurch gekennzeichnet, daß mindestens zwei der Reste Z₁, Z₂ und Z₃ von einem Wasserstoffatom verschieden sind.

4. Verbindungen nach einem der vorstehenden Ansprüche, dadurch gekennzeichnet, daß mindestens einer der Reste Z₁, Z₂ und Z₃ eine Methoxygruppe bilden.

5. Verbindungen nach einem der vorstehenden Ansprüche, dadurch gekennzeichnet, daß die Salze als Additionssalze einer Mineralsäure vorliegen, die unter Schwefelsäure, Salzsäure, Salpetersäure und Phosphorsäure ausgewählt sind.

6. Verbindungen nach einem der Ansprüche 1 bis 4, dadurch gekennzeichnet, daß die Salze als Additionssalze einer Mineralbase oder einer organischen Base vorliegen, die unter Schwefel, Kalium oder Triethanolamin ausgewählt sind.

7. Verbindungen nach einem der Ansprüche 1 bis 4, dadurch gekennzeichnet, daß die Komplexe solche Komplexe darstellen, die durch die Zugabe von Zinkchlorid oder Calciumchlorid gebildet wurden.

8. Verbindungen nach einem der vorstehenden Ansprüche, dadurch gekennzeichnet, daß sie unter den folgenden Verbindungen ausgewählt sind:
N,N'-Bis-(3,4,5-trimethoxybenzyl)-2-methylethylendiamin-N,N'-diessigsäure,
N,N'-Bis-(3,4,5-trimethoxybenzyl)-propylendiamin-N,N'-diessigsäure,
N,N'-Bis-(3,5-di-tert-butyl-4-hydroxybenzyl)-2-methylethylendiamin-N,N'-diessigsäure,
N,N'-Bis-(3,4,5-trimethoxybenzyl)-1,2-cyclohexyldiamin-N,N'-diessigsäure,
Ethyl-N,N'-bis-(3,4,5-trimethoxybenzyl)-ethylendiamin-N,N'-di-(2-methyl)-acetat,
N,N'-Bis-(3-hydroxy-4-methoxybenzyl)-ethylendiamin-N,N'-dipropionsäure,
N-(3,5-Dimethoxybenzyl)-ethylendiamin-N,N',N'-tripropionsäure,
N,N''-Bis-(3,4,5-trimethoxyenzyl)-diethylentriamin-N,N',N''-triessigsäure,
N,N'-Bis-(3,4,5-trimethoxybenzyl)-2-methylethylendiamin-N,N'-dipropionsäure.

9. Pharmazeutische oder kosmetische Zubereitung, dadurch gekennzeichnet, daß sie in einem pharmazeutisch oder kosmetisch annehmbaren Träger mindestens eine Verbindung gemäß der Formel (I), wie sie nach einem der Ansprüche 1 bis 8 beansprucht ist, enthält.

10. Zubereitung nach Anspruch 9, dadurch gekennzeichnet, daß sie die Verbindung gemäß der Formel (I) in einem Mengenverhältnis von 0,001 bis 10 Gew.-% in bezug auf das Gesamtgewicht der Zusammensetzung enthält.

11. Zubereitung nach Anspruch 9 oder 10, dadurch gekennzeichnet, daß sie zusätzlich noch mindestens einen Wirkstoff enthält, der aus Antilipoperoxidantien, biologischen Reduktionsmitteln, Sauerstofffängern, Enzymen, einer Superoxid-Dismutase (SOD) oder eine SOD-ähnliche Substanz, die Glutathioperoxydase oder Systeme auf Basis von Selen enthält.

12. Verwendung einer Verbindung gemaß der Formel (I) nach einem der Ansprüche 1 bis 8 zur Herstellung eines Arzneimittels, das zur Behandlung von Stressbedingungen bestimmt ist, die mit bestimmten pathologischen Zuständen verbunden sind.

13. Verwendung einer Verbindung gemäß der Formel (I) nach einem der Ansprüche 1 bis 8 bei oder zur Herstellung einer kosmetischen oder pharmazeutischen Zubereitung, die zur Verhütung der Hautalterung bestimmt ist.

14. Verwendung einer Verbindung gemäß der Formel (I) nach einem der Ansprüche 1 bis 8 als Antioxidans in pharmazeutischen und kosmetischen Zubereitungen.
